Europäisches Patentamt

European Patent Office

Office européen des brevets

(19)

(11) Publication number: **0 176 352 B1**

# EUROPEAN PATENT SPECIFICATION

(45) Date of publication of patent specification: **17.07.91**

(21) Application number: **85306794.0**

(22) Date of filing: **24.09.85**

(51) Int. Cl.⁵: **G01R 33/32**, G01R 33/50, A61B 5/05

(54) **Difference technique using NMR measurements to screen for cancer in human tissue.**

(30) Priority: **26.09.84 US 654957**

(43) Date of publication of application:
**02.04.86 Bulletin 86/14**

(45) Publication of the grant of the patent:
**17.07.91 Bulletin 91/29**

(84) Designated Contracting States:
**CH DE FR GB IT LI**

(56) References cited:
**EP-A- 0 132 358**
**CA-A- 1 004 297**

(73) Proprietor: **SOUTHWEST RESEARCH INSTI-
TUTE**
**Post Office Drawer 28510 6220 Culebra Road
San Antonio, TX 78284(US)**

(72) Inventor: **Rollwitz, William J.**
**213 Halbart Drive
San Antonio Texas 78213(US)**

(74) Representative: **Adams, William Gordon et al
RAWORTH, MOSS & COOK 36 Sydenham
Road
Croydon Surrey CR0 2EF(GB)**

## Description

Background of the Disclosure

This method is intended to enhance data information obtained by NMR interrogation of human tissue, particularly with a view of locating small tumors. As set forth in a related application US-A-4608991 (EP-A-176353) filed on the same day as the present disclosure, an apparatus is disclosed which enables the sequential segmented interrogation of the human breast with a particular goal of locating relatively small tumors. Enhancement of that data is important to enable small tumors to be located. It is assumed that large or gross tumors can be located by other procedures. However, early detection enhances the chance of recovery. Early detection enables less radical curative procedures to be utilized.

Smaller tumors yield smaller signals. This NMR procedure thus will locate tumors with signals that are quite small. An example of NMR application to cancer screening is shown in CA-A-1004297 (US-A-3789832).

The method of claim 1 of this application enhances the signal component traceable to the tumor, reducing the other signal components and thereby focusing on the signal component derivative from prospective tumors. In that manner, signal levels above the millivolt range can thus be obtained, and are therefore more readily located in the NMR signal to enable the output signal to be processed. This reduces the size of the minimum tumor which can be located through the procedure and method of this application.

Brief Description of the Drawings

So that the manner in which the above recited features, advantages and objects of the present invention are attained and can be understood in detail, more particular description of the invention, briefly summarized above, may be had by reference to the embodiments thereof which are illustrated in the appended drawings.

It is to be noted, however, that the appended drawings illustrate only typical embodiments of this invention and are therefore not to be considered limiting of its scope, for the invention may admit to other equally effective embodiments.

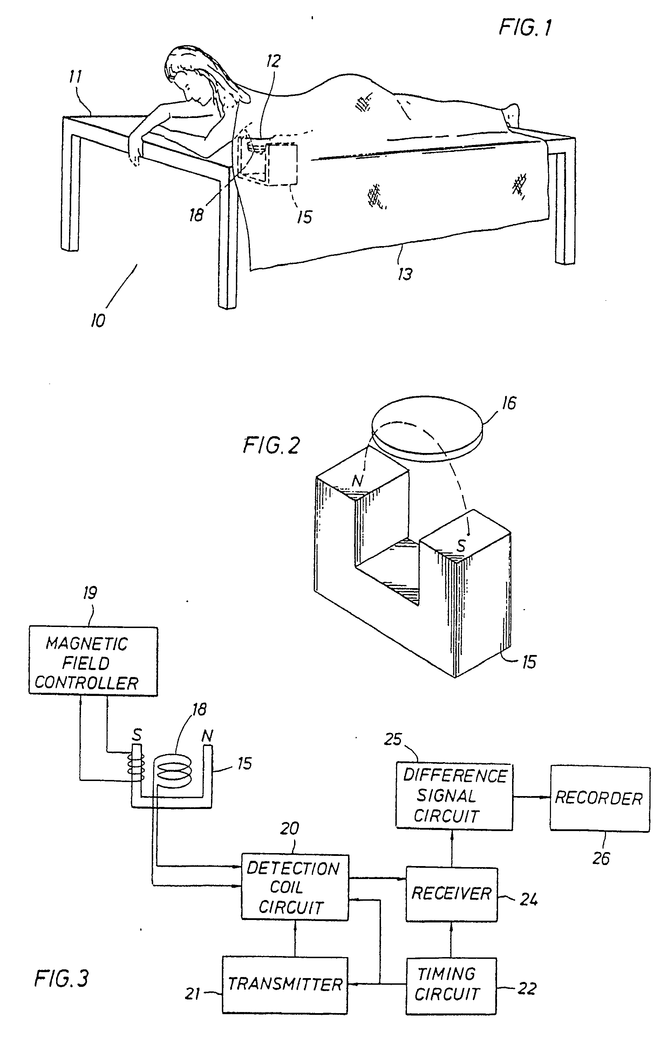

Fig. 1 shows a patient undergoing examination with the NMR breast test apparatus of the present disclosure;

Fig. 2 is a perspective view of a magnet forming a magnetic field having a field intensity defining a sensitive volume undergoing tests;

Fig. 3 is a schematic block diagram of the NMR test circuitry;

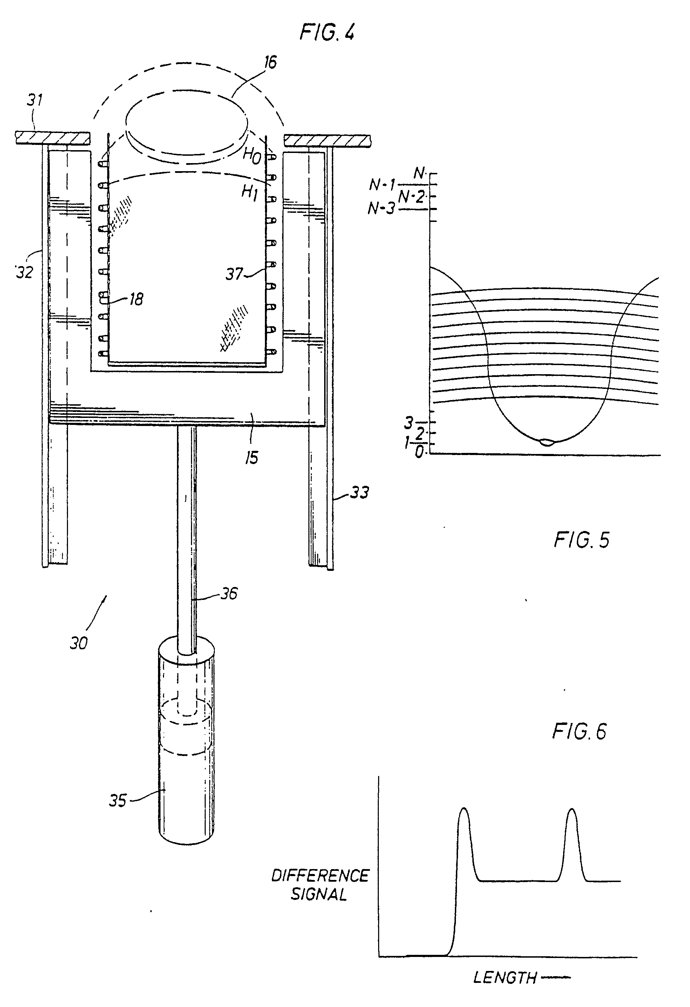

Fig. 4 shows one form of apparatus moving a magnetic field gradient so that the sensitive volume is moved over a period of time to examine the entire breast;

Fig. 5 is a side view of a pendulant breast showing segments of body tissue examined on different sequential tests interrogations; and

Fig. 6 is a graph of the difference signal as a function of breast length.

Detailed Description of the Preferred Procedure

This procedure contemplates the successive NMR interrogation of slices of the human tissues, particularly the female breast for the express purpose of obtaining individual signals from each slice. So to speak, a specified thickness is determined by the magnetic field gradient to thereby segment the female breast into a number of slices. Each interrogation NMR procedure obtains an output signal for each slice. The magnetic field is changed, relocating the magnetic field intensity $H_0$ requisite to obtain a proper NMR response. Assume for purposes of discussion and description that this slice is a thickness that is more or less uniform from slice to slice and is approximately one millimeter. This is a reasonable scale factor for the procedure described herein. That is, the slice of space within the magnetic field where the field intensity is $H_0$ is about one millimeter thick. This slice is then relocated from test point to test point. Preferably, the slices overlap slightly. If they do not overlap, there is the possibility that a certain portion of the female breast will be overlooked and that a tumor in that area will not be observed. It is preferable to have a degree of overlap, perhaps as much as 50%. The degree of overlap is therefore typically 25%-75%. Each slice is preferably thin and typically in the range of the preferred dimension of about one millimeter. This can also be varied and is dependent on a number of factors including scale factors.

The above mentioned application sets forth an apparatus described in the attached drawings, and that specification is hereby incorporated by reference. That apparatus is used to obtain successive data from NMR interrogations. Each interrogation up to N interrogations is an NMR output. Each data output is utilized in detection of cancer tumors.

This procedure detects hydrogen which is in the water in the various tissues undergoing examination. Water in the tissues is held by different binding mechanisms or binding phases. The binding varies and hence, the NMR responses will vary. The portion of water may vary with binding mechanisms. Such binding changes are variant. For definition, permit the fraction of water present in phase number i be denoted by $P_i$ (typically a fraction or percent of water). For water in the phase i the relaxation time for that water is $T_i$. Assume that there is a slow exchange between phases, an assumption that the relaxation time of this phase $T_i$ is less than the lifetime of water in phase i, the NMR signal voltage as a function of time is given by Equation (1):

$$(1) \quad v(t) = \sum_{i=1}^{n} P_i e^{\frac{-t}{T_i}}$$

Recalling that there are up to n phases of water present, each particular phase of water binding relaxes after NMR interrogation independent of the others and each has a time constant $T_i$ through $T_n$ respectively. Equation (1) was based on a slow exchange. In the event of a rapid exchange wherein $T_i$ is much greater than the lifetime in phase i, the NMR signal voltage as a function of time is given by Equation (2):

$$(2) \quad v(t) = \exp\left[-t / \left(\sum_{i=1}^{n} P_i / T_i\right)\right]$$

Summing Equation (2) for all water phases where $i = 1$ up to $i = n$, one obtains a single characteristic time given by Equation (3):

$$(3) \quad \frac{1}{T} = \sum_{i=1}^{n} \frac{P_i}{T_i}$$

Simplifications can be made from the general statements given above by reducing n to two, the typical case involving NMR interrogation of the female breast in detection of water which is bound in normal tissue. This permits development of a relatively exact expression. This exact expression relates to tissue water which can be generally described as tightly bound and loosely bound water. In the presence of a cancerous tumor, the relaxation time for water in the cancerous tissue is longer than the relaxation time for the healthy tissue. Because the tissue is made of water in various binding phases, and because there is exchange between the two phases of water in both healthy tissue and cancerous tissue, the NMR signal is given either by Equation (1) for slow water exchange or by Equations (2) and (3) for a rapid water exchange.

Assume that the female breast includes a cancer. Assume that a slow interchange of water does occur between the healthy tissue and the cancerous tissue. The water will then be in three phases: one tightly bound, one loosely bound in the healthy tissue, and a third loosely bound in the cancer site. Under this assumption, $i = 3$ for Equation (1) and hence, Equation (1) can be written as Equation (4):

$$(4) \quad v_2(t) = P_{21} e^{\frac{-t}{T_{21}}} + P_{22} e^{\frac{-t}{T_{22}}} + P_{23} e^{\frac{-t}{T_{23}}}$$

In Equation (4), the fractions P are the fractions of water in each binding phase converted into voltages. Likewise, each water phase has its own characterisic spin-spin relaxation time $T_2$ thereafter and this is also shown in Equation (4). Equation (4) is an equation for the spin-spin relaxation time signal which is $v_2(t)$ while Equation (5) can be written for the spin-lattice relaxation time $v_1(t)$. This equation follows the same general form as Equation (4).

$$(5) \quad v_1(t) = P_{11}e^{\frac{-t}{T_{11}}} + P_{12}e^{\frac{-t}{T_{12}}} + P_{13}e^{\frac{-t}{T_{13}}}$$

Assuming water in healthy tissue is in two binding phases, the hydrogen transient NMR signal is given by Equation (6):

$$(6) \quad v_{h2}(t) = P_{21h}e^{\frac{-t}{T_{21}}} + P_{22h}e^{\frac{-t}{T_{22}}}$$

In similar fashion, the hydrogen transient NMR system involving a three component system where one component is cancerous tissue and the other two components are healthy tissue is given by Equation (7):

$$(7) \quad v_{c2}(t) = P_{21c}e^{\frac{-t}{T_{21}}} + P_{22c}e^{\frac{-t}{T_{22}}} + P_{23c}e^{\frac{-t}{T_{23}}}$$

Assume as successive NMR signals are obtained from adjacent layers through the female breast (a progression from the first to the Nth layer), the signal of Equation (6) represents a signal from a layer of healthy tissue while the signal of Equation (7) represents a layer having cancerous tissue. Subtracting Equation (6) from Equation (7) yields a difference signal given by Equation (8):

$$(8) \quad v_{diff.} = v_{c2} - v_{h2}$$

Grouping similar relaxation times, Equation (8) can then be written as Equation (9) where the difference signal is given by:

$$(9) \quad v_{diff.} = (P_{21c} - P_{21h})e^{\frac{-t}{T_{21}}} + (P_{22c} - P_{22h})e^{\frac{-t}{T_{22}}} + P_{23c}e^{\frac{-t}{T_{23}}}$$

The first and second terms of Equation (9) have similar coefficients and hence their difference becomes relatively small. That is, they are relatively small, observing the subtractive factors. By contrast, the third term is by its nature a small coefficient. A limit condition can be obtained as the cancerous tissue approaches zero or all tissue becomes healthy. In that event, the approach toward zero impacts each of the subtractive coefficients of the first and second terms where the limit is given by $P_{21c} = P_{21h}$. This reduces the first term toward zero. In like fashion, the limit observed at $P_{22c} = P_{22h}$ substantially reduces the second term. In these limit conditions, both the first and second terms drop out, yielding Equation (10) which is markedly simpler in contrast with Equation (9):

$$(10) \quad v_{diff.} \ (limit) = P_{23c}e^{\frac{-t}{T_{23}}}$$

To obtain Equation (10), it will be observed that certain limits are approached. These limits reduce the signal resulting from similarities while accenting or increasing the relative difference (see Equation 10). In the limit situation, the difference signal contains only the cancerous component. The difference signal, showing decreased similarities and increased differences, provides an accent between adjacent layers from sequential NMR hydrogen transient signals. A graph of the difference signals (between adjacent layers) as a function of layers (as the number of layers approaches N) yields an indication of the first and last layers in which the cancerous tumor is located. That is, assume that the tumor is located between layers 100 and 105. If 150 data points are taken (N = 150), then the difference signal will not yield an image indicative of a cancerous tumor until N is in the range of 100-105. This helps isolate the layer(s) in which the tumor is located. The difference signal can thus be isolated and the relative location of the tumor is then known.

Utilizing typical scale factors, the NMR signal level is in the range of 5 volts. In that range, assume that the noise level is around 1 millivolt. Assume further that signals from adjacent layers represent volumes which do not differ by more than about 5%. Geometrically speaking, with 150 samples (N = 150) the shape of the female breast may be approximated from layer to layer as a triangle where the layers will differ by 5% or less in volume between adjacent layers. That is, adjacent layers will not differ in size by more than by 5%. Assuming a digitized signal format (that is, the variable signal is in digital form) and further assuming subtraction accuracy of one part in 1024 parts, the accuracy of the data can then be determined. If a first layer has a 5 volt signal, then the adjacent layer will have a signal of 5.25 volts maximum (recalling the assumption of not more than 5% volumetric variation), then the subtraction of these two signals will provide a resultant output differential of up to 0.25 volts; digitizing accuracy should be added and this typically represents about 0.01 differential. Thus, a normal signal representing up to 5% size change (without cancerous tissue) at the most will be about 0.25 volts ±0.15 volts.

The foregoing is assumed for healthy tissue. Assume that a cancer is present and represents about 1% of the volume. In this event, 1% of the 5 volt signal is about 0.05 volts. A signal level of about 0.05 volts (indicative of cancer) when confronted with a variation of 0.25 plus or minus 0.01 volt as a result of digitizing and geometric change on subtraction yields a cancerous tissue signal which is about 5/30 or 16% of the output differential signal. The signal to noise ratio of the subtracted signal is around 30 to 1. Going now to Equations (6) and (7) and making assumptions based on the above geometry (a digitizing error of 0.01 volt, a geometric factor of 5%, and a desire to locate cancers of 1% of the volume of the layer), this then provides values for the constants in Equation (6) and (7). The constants are:

$P_{21h}$ = 5/3

$P_{22h}$ = 10/3

$P_{21c}$ = 5.25/3

$P_{22c}$ = 10.5/3

$P_{23c}$ = 0.05

Substituting these values into Equations (6) and (7), the values obtained from Equation (9) for the first and second coefficients are:

$$(11) \quad P_{21c} - P_{22h} = \frac{0.25}{3}$$

$$(12) \quad P_{22c} - P_{22h} = \frac{0.5}{3}$$

Substituting these values from Equations (11) and (12) into Equation (9), the difference equation then becomes:

$$(13) \quad v_{diff.} = \frac{0.25}{3}e^{\frac{-t}{T_{21}}} + \frac{0.5}{3}e^{\frac{-t}{T_{22}}} + 0.05e^{\frac{-t}{T_{23}}}$$

5

Setting the exponentials to a time equal to zero obtains Equation (14):

$$(14) \quad v_{diff.} (t=0) = 0.25 \pm 0.01 + 0.05 = 0.30 \pm 0.01$$

From the foregoing, it will be observed that the assumption of a cancer filling 1% of the volume of the slice of the female breast in comparison with the adjacent slice provides an adequate size signal with a suitable signal to noise ratio to enable detection of the cancer output signal. That is, this cancer signal is sufficiently large to enable detection of a signal of this small amplitude. It is generally believed that detection of cancerous tumors in the size of 1% volume of a particular slice is sufficiently sensitive and represents such early detection that curative procedures can be more readily adapted.

The manner of presentation of this data should be considered. Assume that N = 150. Assume also that the time required to obtain each data point is one second, a relatively slow repetition rate. 150 data points can be obtained. This yields 149 difference signals. The signals are obtained by subtracting consecutive or adjacent data entries as N approaches 150. The presentation of data is perhaps best implemented on a white to gray scale on an oscilloscope presentation. The difference signal from adjacent pixels enhances the differences as described above so that the enhanced image will more readily show cancerous tissue.

Therefore, the location of the cancer can then be readily determined. Consider as an extreme example a cancer which represents 10% of a given slice or layer. In that event, signal amplitude is markedly increased and a signal to noise ratio is improved even further.

For the sake of good health, 1% volume seems a minimum value for cancer detection. The signal from healthy breast tissue may have two parts, one from the hydrogen in the tissue and one from the hydrogen in the water. When cancerous tissue is present (along with healthy tissue) there are two values of $T_2$ for water, one for the water in the healthy tissue $T_{2h}$ and a second for the water in the cancerous tissue $T_{2c}$. It has been found experimentally, on the average, that $T_{2c}$ equals $2T_{2h}$. The echo signal from a thin layer of a breast with cancerous tissue will have a voltage amplitude given by Equation (15):

$$(15) \quad a_1 = A_{11} \exp. \frac{-t}{T_{2h}} + A_{21} \exp. \frac{-t}{T_{2c}}$$

where $A_{11}$ is a voltage proportional to the number of hydrogen nuclei in the volume of the water in the healthy tissue, and $A_{21}$ is the voltage proportional to the number of hydrogen nuclei in the volume of the water in the cancerous tissue. By definition, the total volume of the layer of breast is $A_{01} = A_{11} + A_{21}$. When Equation (15) is normalized to unity at t equals zero, one obtains Equation (16):

$$(16) \quad \frac{a_1}{A_{01}} = \frac{A_{11}}{A_{01}} \exp. \frac{-t}{T_{2h}} + \frac{A_{21}}{A_{01}} \exp. \frac{-t}{T_{2c}}$$

Targetting a mimimum size of 1.0% for $A_{21}$ of the total volume, then Equation (17) is:

$$(17) \quad \frac{a_1}{A_{01}} = 0.99 \exp. \frac{-t}{T_{2h}} + 0.01 \exp. \frac{-t}{T_{2c}}$$

When the NMR measurements are made on N layers through the breast, there will be somewhere a layer (n) without cancer adjacent to the layer (n-1) with cancer. The equation for the n layer without cancer, follows Equation (16), will be Equation (18):

$$(18) \quad \frac{a_2}{A_{02}} = \frac{A_{12}}{A_{02}} \exp. \frac{-t}{T_{2h}}$$

where $A_{12}$ is a voltage proportional to the number of hydrogen nuclei in the water in the volume of the n layer without cancer adjacent to n-1 layer.

This difference technique involves the subtraction of Equation (18) from Equation (16). First assume that the volumes of the water in the two adjacent layers of breast are equal and the tissue density is the same, that is $A_{01} = A_{02}$. When Equation (18) is subtracted from Equation (17) the result is Equation (19):

$$(19) \quad a_{diff} = \frac{a_1}{A_{01}} - \frac{a_2}{A_{02}} = 0.01 \exp. \frac{-t}{T_{2h}} + 0.01 \exp. \frac{-t}{T_{2c}}$$

with equal volumes in adjacent layers (n and n-1), the healthy component equals the cancer component. If it is assumed that the volume of the n-1 layer slice with the cancer is up to 5% greater than the layer without the cancer, then from Equation (18) one obtains Equation (20):

$$(20) \quad \frac{a_2}{A_{02}} = 0.94 \exp. \frac{-t}{T_{2h}}$$

When Equation (20) is subtracted from Equation (17), or when the signals from adjacent layers are subtracted, the result is Equation (21):

$$(21) \quad a_{diff} = \frac{a_1}{A_{01}} - \frac{a_2}{A_{02}} = 0.05 \exp. \frac{-t}{T_{2h}} + 0.01 \exp. \frac{-t}{T_{2c}}$$

In Equation (21), the ratio of the two signal components is now 1/5 whereas before subtraction they were 1/99 in Equation (17). The values of $T_{2h}$ and $T_{2c}$ given in the literature are 0.05 and 0.10 seconds respectively. When these values are used, Equation (17) gives a straight-line to graph beyond 0.7 second where the cancerous tissue signal component becomes readily apparent. The difference signal of Equation (21) gives a graph with the cancer component evident beyond 0.3 second because the cancer signal component is then larger.

NMR interrogation is not limited to a single pulse; the interrogation pulse sequence can be a dual pulse sequence with specific spacing between the two pulses. The voltage amplitude of the echo following a 90° - 180° dual-pulse sequence is directly proportional to $M_o$, the nuclear magnetization, and to an exponential with a time constant is $T_2$. Equation (22) is:

$$(22) \quad v_{echo} = \frac{1}{2} k_1 M_o \exp. (\frac{-2\tau}{T_2})$$

where $\tau$ is the spacing between the 90° and 180° pulses and $k_1$ is the detection constant. The nuclear magnetization $M_o$ is $X_n H_o$ where $X_n$ is the nuclear volume susceptibility and $H_o$ is the applied magnetic field. Substituting the nuclear volume susceptibility definition, the nuclear magnetization is Equation (23):

$$(23) \quad M_o = \frac{N_o \left| \mu \right|^2 H_o}{3kT}$$

where $N_o$ is the total number of nuclei per unit volume, $\mu$ is the nuclear magnetic moment, k is the Boltzmann constant and T is the absolute temperature. When Equation (23) is substituted into Equation (22), the echo voltage amplitude is Equation (24):

$$(24) \quad v_{echo} = \frac{1}{2} k_1 \left( \frac{N_o \left| \mu \right|^2 H_o}{3kT} \right) \exp. \left( \frac{-2\tau}{T_2} \right)$$

thus it is seen that the echo voltage is directly proportional to the number of detected nuclei per unit volume.

The echo voltage output signal is verification that tumor mass is proportional to signal amplitude and hence signal size. This is significant in establishing minimum tumor size for NMR detection. Consider the minimum size tumor which can be detected using this technique. Assume that the N layers of the breast tested by this procedure are right cylinders and hence have a volume of $\pi r^2 d$ where r is radius and thickness is represented by d. A small cancer with a cross-sectional area of 0.78 cm$^2$ has a volume of the cancer in the layer of 0.78d cm$^3$. The layer volume then is 78d while the cancer volume is 0.78d, making the cancer to be one percent of the layer volume if r is assumed to be 5 cm. In this instance, a cancer of this size is significantly small that early detection markedly increases the probabilities of full health protection.

At Equation (6) and following, water in both healthy and cancerous tissue was assumed. The hydrogen NMR response from both types of tissue is the talisman yielding NMR response for detection. Water in tissue is in differing binding phases. There is exchange between these phases, and, in general, if $P_i$ denotes the fraction of water present in phase number i, if the relaxation time for this phase is $T_i$, if there is a slow exchange ($T_i \ll$ the lifetime in phase number i), then the NMR signal voltage as a function of time is Equation (25):

$$(25) \quad v(t) = \sum_{i=t}^{n} P_i e^{\frac{-t}{T_i}}$$

Each phase relaxes essentially independently with its own time constant $T_i$. For the case of rapid exchange ($T_i \gg$ lifetime in phase number i), the NMR signal voltage as a function of time is Equation (26):

$$(26) \quad v(t) = \exp \left( -t / \left( \sum_{i=1}^{n} P_i / T_i \right) \right)$$

For rapid exchange, the whole system relaxes with a single characteristic time T given by Equation (27):

$$(27) \quad \frac{1}{T} = \sum_{i=1}^{n} \frac{P_i}{T_i}$$

In the special case of two phases, an exact expression can be developed for the condition between the slow and the fast exchange limiting cases.

Most of the measurements of NMR relaxation data from tissue water have shown two phases that are sometimes known as "bound" and "free". When cancer is present, the relaxation time for the water in the cancerous tissue is twice the relaxation time in the healthy tissue. When there is a slow exchange betweeen the water in the healthy and cancerous tissue, then the NMR signal will be acccording to Equation (25). When there is a rapid exchange, the NMR signal will follow Equations (26) and (27).

It has been shown that there are two relaxation times (slow interaction) for the water in cancer-containing tissue. There is one relaxation from the hydrogen in the water in the healthy tissue and another from the hydrogen in the water in the cancerous tissue. For this case, Equation (25) can be written for $i = 2$ to be the form of Equation (6) above. From that equation, the fractions $P_{21}$ and $P_{22}$ are the fractions in each binding phase of the water in healthy tissue, and $T_{21}$ and $T_{22}$ are the values of the spin-spin relaxation time for each water phase.

The hydrogen transient NMR signal from healthy tissue with one water component is Equation (28):

$$(28) \quad v_{h2}(t) = P_{21h}e^{\frac{-t}{T_{21}}}$$

and that from cancerous tissue with two water binding components (simplified from Equation 7) is given by Equation (29):

$$(29) \quad v_{c2}(t) = P_{21c}e^{\frac{-t}{T_{21}}} + P_{22c}e^{\frac{-t}{T_{22}}}$$

In this difference technique, the NMR signal of Equation (28) is from the layer of tissue adjacent to the cancerous layer giving the NMR signal of Equation (29), and the equations are subtracted to obtain a difference signal or $v_{c2} - v_{h2}$. Considering that $T_{21}$ has the same value in Equations (28) and (29), the difference signal is Equation (30):

$$(30) \quad v_{diff.} = (P_{21c} - P_{21h})e^{\frac{-t}{T_{21}}} + P_{22c}e^{\frac{-t}{T_{22}}}$$

This difference signal (similar to the three phase relationship in Equation 9) gives a decreased amplitude for the $T_{21}$ term, but the component from the cancerous tissue is not decreased. In the limit case of $P_{21c} = P_{21h}$, the difference signal is similar to Equation (10) and is Equation (31):

$$(31) \quad v_{diff.}(limit) = P_{22c}e^{\frac{-t}{T_{22}}}$$

Considering Equations (30) and (31), the difference signal decreases the similarities between the hydrogen transient NMR signals from healthy and cancerous tissue while increasing the relative difference between them. In the limit where the similarities are the same, the difference signal will contain only the cancerous component.

The use of the difference signal, because it decreases similarities and increases differences, turns the hydrogen transient NMR signals from layers of tissue into displays or the differences between adjacent layers. The graph of the difference signals between adjacent layers, as a function of location from O to N layers gives an indication of the beginning and ending of a cancerous inclusion in one or more layers. The difference signal will not give an image of the cancerous inclusion but will indicate in which layer or layers it resides for an enchanced screening indication. This difference indication is much less complicated and less expensive for tumor screening.

9

**Claims**

1. A method of conducting a non-invasive female breast cancer test comprising the steps of:

   (a) forming a magnetic field between the poles of a magnet wherein the magnetic field defines a sensitive volume between the poles and the sensitive volume has the requisite magnetic field intensity for NMR testing;

   (b) moving incrementally from a beginning point toward an ending point the sensitive volume to scan a female breast;

   (c) periodically interrogating by a transmitted pulse from a coil and the breast portion located in the sensitive volume for NMR response;

   (d) wherein the magnetic field intensity in the sensitive volume and the pulse from the coil cause an NMR response from the breast portion; and

   (e) determining anomalies arising from abnormal growths as indicated by variations in NMR responses from different pulse interrogations.

2. The method of Claim 1 wherein the different pulse interrogations are for adjacent breast portions on incrementally moving the sensitive volume.

3. The method of Claim 2 wherein consecutive NMR responses are subtracted to obtain a difference signal for different and adjacent breast portions.

4. The method of Claim 1 wherein the testing is for hydrogen NMR response, and wherein the hydrogen has differing binding phases in cancerous tissue compared to normal tissue, and said method obtains NMR responses for adjacent breast portions which responses are subtracted from one another to yield a difference signal having a cancer tissue binding phase compound.

5. The method of Claim 4 wherein healthy tissue response tends to null on subtraction.

6. The method of Claim 5 wherein healthy tissue water binding phase has a common component in the response of two adjacent breast portion interrogations, and said common components are approximately equal for subtraction.

7. The method of Claim 1 wherein adjacent breast portion responses are subtracted to obtain a difference signal having a $T_1$ component.

8. The method of Claim 1 wherein adjacent breast portion responses are subtracted to obtain a difference signal having a $T_2$ component.

9. The method of Claim 1 wherein the NMR response is obtained from hydrogen in water and the water in the breast tissue is relatively tightly or relatively loosely bound, and wherein the relaxation time for water in cancerous tissue is longer, the method including the step of observing anomalies indicative of cancer by comparing signals from consecutive and adjacent sensitive volumes of the female breast.

10. The method of Claim 1 including the step of obtaining NMR measurements from adjacent breast portions wherein the adjacent portions have a relative volume within 5% of one another, all as obtained by hanging with the aid of gravity the female breast in the magnetic field, thereby obtaining a pendulous breast, and different signal are obtained therefrom indicative of variations in relaxation time of hydrogen in the water in the cancerous tissue in contrast with healthy tissue.

11. The method of Claim 1 wherein the interrogation relies on the FID response of the body tissue, the FID response following a dual pulse interrogation sequence.

12. The method of Claim 11 wherein the interrogation involves a dual pulse interrogation having timed spacing of $90°\text{-}\tau\text{-}180°$.

13. The method of Claim 1 wherein the NMR response is obtained from hydrogen in water in the tissue, the water being found in the healthy tissue and in cancerous tissue, and NMR responses are obtained from adjacent segmented portions of the female breast, the method including the step of subtracting

10

output signals of adjacent breast portions to obtain a different signal at limit which is proportionate to $Ke^{-t/T}$ where K is a constant, where t is elapsed time, where T is the relaxation time for water in the breast.

## Revendications

1. Un procédé pour effectuer, sans pénétration, une analyse du cancer féminin du sein comprenant les étapes consistant à :

   (a) former un champ magnétique entre les pôles d'un aimant où le champ magnétique définit un volume sensible entre les pôles, et le volume sensible possède l'intensité de champ magnétique pour l'analyse par résonance magnétique nucléaire (ou RMN);

   (b) déplacer par incréments, à partir d'un point d'origine vers un point final, le volume sensible pour balayer un sein féminin;

   (c) interroger périodiquement par une impulsion transmise depuis une bobine et la partie de sein située dans le volume sensible en vue d'une réponse RMN;

   (d) dans lequel l'intensité de champ magnétique dans le volume sensible et l'impulsion provenant de la bobine provoquent une réponse RMN de la partie de sein; et

   (e) déterminer des anomalies provenant de croissances anormales telles qu'elles sont indiquées par des variations des réponses RMN provenant de différentes interrogations d'impulsions.

2. Le procédé selon la revendication 1 dans lequel les différentes interrogations par impulsions concernent des parties adjacentes du sein lors d'un déplacement par incréments du volume sensible.

3. Le procédé selon la revendication 2 dans lequel des réponses consécutives RMN sont soustraites pour obtenir un signal de différence pour des parties de sein différentes et adjacentes.

4. Le procédé selon la revendication 1 dans lequel l'analyse concerne une réponse RMN en hydrogène et dans lequel l'hydrogène possède, dans un tissu cancéreux, des phases de liaisons différentes de celles d'un tissu normal, et ledit procédé obtient des réponses RMN pour des parties adjacentes de sein, ces réponses étant soustraites l'une de l'autre pour donner un signal de différence qui possède une composante de phase reliée à un tissu cancéreux.

5. Le procédé selon la revendication 4 dans lequel la réponse d'un tissu sain tend vers zéro lors de la soustraction.

6. Le procédé selon la revendication 5 dans lequel la phase de liaison d'eau d'un tissu sain comporte une composante commune dans la réponse de deux interrogations de parties adjacentes de sein, et lesdites composantes communes sont à peu près égales en vue d'une soustraction.

7. Le procédé selon la revendication 1 dans lequel des réponses de parties adjacentes du sein sont soustraites pour obtenir un signal de différence comportant une composante $T_1$.

8. Le procédé selon la revendication 1 dans lequel des réponses de parties adjacentes de sein sont soustraites pour obtenir un signal de différence comportant une composante $T_2$.

9. Un procédé selon la revendication 1 dans lequel la réponse RMN est obtenue à partir de l'hydrogène de l'eau et l'eau dans le tissu du sein est liée de façon relativement étroite ou de façon relativement lâche, et dans lequel le temps de relâchement de l'eau d'un tissu cancéreux est plus long, le procédé comprenant l'étape consistant à observer des anomalies indicatives du cancer en comparant des signaux provenant de volumes sensibles consécutifs et adjacents du sein féminin.

10. Le procédé selon la revendication 1 comprenant l'étape consistant à obtenir, pour des parties adjacentes de sein où les volumes relatifs de parties adjacentes diffèrent de moins de 5 % l'un de l'autre, des mesures RMN toutes obtenues en suspendant par la pesanteur le sein féminin dans le champ magnétique, en obtenant ainsi un sein en pendule, et des signaux différents, indicatifs de variations du temps de relâchement de l'hydrogène dans l'eau des tissus cancéreux par rapport aux tissus sains,sont obtenus à partir de ces mesures.

11. Le procédé selon la revendication 1 dans lequel l'interrogation repose sur la réponse de détecteur d'ionisation de flamme (ou DIF) du tissu du corps, la réponse DIF suivant une séquence d'interrogation à double impulsion.

12. Le procédé selon la revendication 11 dans lequel l'interrogation implique une interrogation à double impulsion avec des intervalles de temps de 90° à 180°.

13. Le procédé selon la revendication 1 dans lequel la réponse RMN est obtenue à partir de l'hydrogène de l'eau du tissu, l'eau étant trouvée dans le tissu sain et dans le tissu cancéreux, et des réponses RMN sont obtenues à partir de parties adjacentes segmentées du sein féminin, le procédé comprenant l'étape consistant à soustraire des signaux de sortie de parties adjacentes du sein pour obtenir un signal différent à la limite qui est proportionnel à $Ke^{-t/T}$ où K est une constante, t est le temps écoulé, T est le temps de relaxation de l'eau dans le sein.

## Patentansprüche

1. Ein Verfahren zur Durchführung eines ohne Eingriff durchzuführenden Frauenbrustkrebs-Testes, das folgende Schritte umfaßt:
a) Aufbauen eines Magnetfeldes zwischen den Polen eines Magneten, wobei das Magnetfeld ein sensitives Volumen zwischen den Polen definiert und das sensitive Volumen die erforderliche magnetische Feldstärke für eine NMR-Untersuchung (Untersuchung mittels magnetischer Kernresonanz) aufweist;
b) inkrementales Bewegen von einem Anfangspunkt zu einem Endpunkt des sensitiven Volumens, um eine Frauenbrust abzutasten;
c) periodisches Abfragen nach einer NMR-Reaktion durch einen übertragenen Impuls von einer Spule und der in dem sensitiven Volumen angeordneten Brustpartie;
d) wobei die magnetische Feldstärke in dem sensitiven Volumen und der Impuls von der Spule eine NMR-Reaktion von der Brustpartie bewirken; und
e) Bestimmung von Anomalien, die sich aus anormalem Wachstum ergeben, die durch Veränderungen in der NMR-Reaktionen von unterschiedlichen Impulsabfragen aufgezeigt werden.

2. Verfahren nach Anspruch 1, bei den die unterschiedlichen Impulsabfragen beim inkrementalen Bewegen des sensitiven Volumens benachbarte Brustpartien betreffen.

3. Verfahren nach Anspruch 2, bei den aneinanderfolgende NMR-Reaktion voneinander abgezogen werden, um ein Differenzsignal für unterschiedliche und benachbarte Brustpartien zu erhalten.

4. Verfahren nach Anspruch 1, bei der die Untersuchung sich auf eine Wasserstoff-NMR-Reaktion richtet und wobei der Wasserstoff unterschiedliche Bindungsphasen im Krebsgewebe verglichen mit dem normalen Gewebe aufweist und wobei die Methode NMR-Reaktionen für benachbarte Brustpartien erzielt, welche Reaktionen, voneinander abgezogen, ein Differenzsignal hervorbringen, das eine Krebsgewebebindungsphasen-Zusammensetzung aufweist.

5. Verfahren nach Anspruch 4, bei der eine Reaktion für Gewebe nach der Subtraktion nach Null tendiert.

6. Verfahren nach Anspruch 5, bei dem die Wasserbindungsphase eines gesunden Gewebes eine gemeinsame Komponente in der Reaktion von Abfragen von zwei benachbarten Brustpartien ist und die gemeinsamen Komponenten bei der Subtraktion ungefähr gleich sind.

7. Verfahren nach Anspruch 1, bei dem Reaktionen von benachbarten Brustpartien voneinander subtrahiert ein Differenzsignal ergeben, das eine $T_1$-Komponente aufweist.

8. Verfahren nach Anspruch 1, bei dem Reaktionen von benachbarten Brustpartien voneinander subtrahiert ein Differenzsignal ergeben, das eine $T_2$-Komponente aufweist.

9. Verfahren nach Anspruch 1, bei dem die NMR-Reaktionen aus Wasserstoff im Wasser erhalten wird und das Wasser in dem Brustgewebe relativ fest oder relativ lose gebunden ist und bei der die Relaxationszeit für Wasser im Krebsgewebe länger ist, wobei das Verfahren Schritte zur Beobachtung

EP 0 176 352 B1

von krebsanzeigenden Anomalien durch Vergleich von Signalen von aufeinanderfolgenden und benachbarten sensitiven Volumina der Frauenbrust enthält.

10. Verfahren nach Anspruch 1, das den Schritt enthält, NMR-Messungen von benachbarten Brustpartien zu erhalten, bei der die benachbarten Partien ein relatives Volumen von 5% voneinander aufweisen, alles dadurch erhalten, daß die Frauenbrust mit Hilfe der Schwerkraft in einem Magnetfeld hängt, wodurch eine pendelartige Brust erhalten wird, und woraus sich unterschiedliche Signale ergeben, die Veränderungen in der Relaxationszeit von Wasserstoff in dem Wasser im Krebsgewebe im Gegensatz zu gesundem Gewebe anzeigen.

11. Verfahren nach Anspruch 1, bei der sich die Abfrage auf die FID-Reaktion des Körpergewebes bezieht, wobei die FID-Reaktion von einer Doppelimpulsabfrage-Sequenz folgt.

12. Verfahren nach Anspruch 11, bei dem die Abfrage eine Doppelimpulsabfrage umfaßt, welche einen zeitlichen Abstand von $90° - \pi - 180°$ aufweist.

13. Verfahren nach Anspruch 1, bei der NMR-Reaktionen durch Wasserstoff im Wasser des Gewebes erhalten werden, wobei das Wasser in dem gesunden Gewebe und in dem Krebsgewebe vorgefunden wird und NMR-Reaktionen durch benachbarte segmentartige Partien der Frauenbrust erhalten werden, wobei das Verfahren den Schritt enthält, Ausgangssignale von benachbarten Brustpartien zu subtrahieren, um ein unterschiedliches Grenzsignal zu erhalten, welches proportional zu $Ke^{-t/T}$ ist, wobei K eine Konstante, t die verstrichene Zeit und T die Relaxationszeit für Wasser in der Brust ist.

13

FIG.1

FIG.2

FIG.3

MAGNETIC FIELD CONTROLLER

DIFFERENCE SIGNAL CIRCUIT

RECORDER

DETECTION COIL CIRCUIT

RECEIVER

TRANSMITTER

TIMING CIRCUIT

FIG. 4

FIG. 5

FIG. 6

DIFFERENCE
SIGNAL

LENGTH